# EUROPEAN PATENT SPECIFICATION

(11) **EP 1 590 332 B1**
(45) Date of publication and mention of the grant of the patent: **27.04.2011**
(21) Application number: 03815133.8
(22) Date of filing: 08.09.2003
(51) Int. Cl.: C07D 235/18, C07D 235/20

(54) **A PROCESS FOR THE SYNTHESIS OF BISBENZIMIDAZOLES AND THEIR DERIVATIVES.**
VERFAHREN ZUR SYNTHESE DER BISBENZIMIDAZOLEN UND IHREN DERIVATEN.
PROCÉDÉ POUR LA SYNTHÈSE DES BISBENZIMIDAZOLES ET LEURS DÉRIVÉS.

(30) Priority: 09.01.2003 IN DE00322003
(43) Date of publication of application: 02.11.2005
(73) Proprietor: University of Delhi, Delhi 110 007 (IN)
(72) Inventor: JAIN, Akash, Dr. B.R. Ambedkar, New Delhi 110 007 (IN); TAWAR, Urmila, Dr. B.R. Ambedkar, New Delhi 110 007 (IN); CHANDRA, Ramesh, Dr. B.R. Ambedkar, New Delhi 110 007 (IN); DWARAKANATH, B.S., Dr. B.R. Ambedkar, New Delhi 110 007 (IN); CHAUDHURY, N. K., Dr. B.R. Ambedkar, New Delhi 110 007 (IN); Tandon, Vibha, New Delhi 110 007 (IN)
(74) Representative: Thomas, Simon
(86) International application number: PCT/IN2003/000301
(87) International publication number: WO 2004/063170

(56) References cited:
- DATABASE CA [Online] SADAT S. E.: 'Unique binding site for bis-benzimidazoles on transfer RNA', XP002992521 Retrieved from STN Database accession no. 126:258426 & CHEMICAL COMMUNICATIONS no. 4, 1997, CAMBRIDGE, pages 385 - 386
- DATABASE CA [Online] JI YU-HUA: 'Tris-benzimidazole derivatives: design, synthesis and DNA sequence recognition', XP002992522 Retrieved from STN Database accession no. 136:131135 & BIOORGANIC & MEDICINAL CHEMISTRY vol. 9, no. 11, 2001, pages 2905 - 2919
- DATABASE CA [Online] TAWAR U.: 'Influence of phenyl ring disubstitution on bisbenzimidazole and terbenzimidazole cytotoxicity', XP002992523 Retrieved from STN Database accession no. 139:261219 & JOURNAL OF MEDICINAL CHEMISTRY vol. 46, no. 18, 2003, pages 3785 - 3792

## Description

### FIELD OF THE INVENTION

This invention relates to the process for the synthesis of bisbenzimidazoles and its derivations.

### BACKGROUND OF THE INVENTION

The DNA ligands such as bisbenzimidazoles Hoechst 33342 and Hoechst 33258, form strong and non-covalent linkages, with the adenine and thymine rich regions in the minor groove of DNA, significantly altering the chromatin structure [Exp. Cell Res. (1973), 81,474-477; Biochemistry (1990), 29,9029-9039]. The dyes Hoechst 33342 and Hoechst 33258 are frequently used in cytometry to stain chromosomes insitu. [J Histochem. Cytochem(1985),33,333,338]. These two bis-benzimidazole compounds become brightly fluorescent when they bind to DNA For a long time, it has been known that Hoechst 33258 binds specifically to AT-rich sequences in DNA [Biochim. Biophys. Acta 1987, 949, 158-168; J. Mol. Biol. 1987, 197, 257; Biochemistry 1991, 30, 182-189; Biochemistry 1991, 30, 10294-10306; EMBO J. 1992, 11, 225-232]. A variety of NMR and X-ray crystal structures of Hoechst 33258 bound to different oligonucleotide duplexes have been published [Biochemistry (1989) 28, 7849-7859; Nucleic Acids. Res. (1990) 18, 3753-3762; J. Chem. Soc., Chem. Commun. (1991) 1770; Eur. J. Biochem (1993) 211, 437-447; Biochemistry (1991) 30, 11377-11388]. Collectively, these structural studies reveal that the drug fits snugly into the minor groove of the double helix, covering a run of four contiguous AT base pairs. The Hoechst 33258-DNA interaction appear to be stabilized by several H-bonding and van der Waals contacts [Structure 1, 177] but in fact these molecular forces are believed to contribute to overall binding affinity [J. Mol. Biol (1997) 271, 244-257]. The hydrophobic transfer of ligand from solution on to its DNA binding sites is more likely to represent the main driving force for the complex formation [J. Mol. Biol. (1997) 271, 244-257]. Administration of these compounds prior to irradiation afford protection against the formation of primary lesions in the aqueous solutions of DNA as well as in the intact cell nucleus. These DNA ligands have also been observed to reduce the radiation-induced cytogenetic damage and cell death in cell cultures, as well as in whole body irradiated animals [Ind. J. Exp. Biol (1998) 36, 375-384; Br. J. Cancer (1989) 60, 715-721]. However, post-irradiation treatment of cells with these ligands has been observed to enhance cell death *in vitro* [Ind. J. Exp. Biol (1998) 36, 375-384]. Free radical scavenging and quenching of DNA radicals appear to be the mechanisms responsible for protection by Hoechst compounds administered prior to irradiation [Int. J. Radiation Oncol. (1992) 23, 579-586; Radioprotection (1997) 32, C1-89], but its role in enhancing the radiation-induced cell death when administered after irradiation is not clearly understood.
The limitations of these minor groove binding ligands as being mutagenic, clatogenic and cytotoxic because of the DNA lesions caused on account of topoisomerase I inhibition, gene expression alteration and repair inhibition prevent them from being used in humans. Therefore, the development of DNA binding ligands (Minor Groove Binding Ligands particularly) that afford radioprotective effect without significant mutagenicity and cytotoxic effects can play a significant role in biological radiation protection.

J. Med. Chem. (2003), 46, pp. 3785-3792 discloses syntheses for bisbenzimidazoles as radioprotecting agents.
Aust. J. Chem. (1994), 47, 247-262 discloses the synthesis and characterisation of boron-containing bisbenzimidazoles related to the DNA minor groove binder Hoechst 33258.
J. Org. Chem. (1947), 12, 799-804 discloses the synthesis of 5-hydroxy-8-nitroquinoline and derivatives.

### OBJECTS OF THE INVENTION

An object of the invention is to synthesize benzimidazoles and its derivatives.
Further object of this invention is to synthesize benzimidazoles having pharmacological activity.
Yet another object of this invention is to synthesize benzimidazoles DNA ligands which can afford radioprotective effect without significant mutagenicity and cytotoxic effects.

### DETAILED DESCRIPTION OF THE INVENTION

According to this invention there is provided a process for the synthesis of bisbenzimidazoles and its derivations comprising:
(i) reacting 5-chloroaniline with zinc dust and acetic anhydride to produce 5-chloroacetanilide;
(ii) reacting 5-chloracetanilide with HNO₃ to produce 2-nitro-5-chloroacetanilide;
(iii) adding sodium methoxide to 2-nitro-5-chloroacetanilide to produce 2-nitro-5-chloroaniline;
(iv) heating 2-nitro-5-chloroaniline, methyl piperazine, anhydrous K₂CO₃ and dimethyl formamide at 100 to 120 °C to produce a mixture which is cooled by pouring ice and is filtered to obtain 5-(4'-methylpiperazin-1'-yl)-2- nitroaniline;
(v) treating 5-(4'-methylpiperazin-1'-yl)-2-nitroaniline with Pd/C to produce 2-amino-4-(4'-methylpiperazin-1'-yl)aniline;
(vi) refluxing a mixture of 2-amino4-(4'-methylpiperazin-1'-yl)aniline and ethyl-4-amino-3-nitrobenzenecarboximidate hydrochloride in the presence of ethanol/glacial acetic acid to produce 4-[5'-(4"-methylpiperazin-1"-yl)benzimidazol-2'-yl]-2-nitroaniline;
(vii) treating a solution of 4-[5'-(4"-methylpiperazin-1"-yl)benzimidazol-2'-yl]-2-nitroaniline with palladium on carbon to yield 2-amino-4-[5'-(4"-methylpiperazin-1"-yl)benzimidazol-2'-yl]aniline; and
(viii) heating 2-amino-4-[5'-(4"-methylpiperazin-1"-yl)benzimidazol-2'-yl]aniline and 3-4-dimethoxybenzaldehyde using nitrobenzene as a solvent at 110 to 150 °C to produce 5-(4-methylpiperazin-1-yl)-2-[2'-(3,4-dimethoxyphenyl)-5'-benzimidazolyl]benzimidazole (DMA); or
(ix) heating 2-amino-4-[5'-(4"-methylpiperazin-1"-yl)benzimidazol-2'-yl]aniline and 5-formyl-[3-methoxy-4-hydroxy benzimidazole] using nitrobenzene at 110 to 150 °C in the presence of argon to produce 5-(4-methylpiperazine-1-yl)-2-[2'-{2"-(4-hydroxy-3-methoxyphenyl)-5"-benzimidazolyl}-5'-benzimidazolyl]benzimidazole (TBZ).

Further according to the invention, there is provided 5-(4-methylpiperazin-1-yl)-2-[2'-(3,4-dimethoxyphenyl)-5'-benzimidazolyl]benzimidazole (DMA) or 5-(4-methylpiperazine-1-yl)-2-[2'-{2"-(4-hydroxy-3-methoxyphenyl)-5"-benzimidazolyl}-5'-benzimidazolyl]benzimidazole (TBZ) for use as a radioprotecting agent.

Also according to the invention, there is provided the use of 5-(4-methylpiperazin-1-yl)-2-[2'-(3,4-dimethoxyphenyl)-5'-benzimidazolyl]benzimidazole (DMA) or 5-(4-methylpiperazine-1-yl)-2-[2'-{2"-(4-hydroxy-3-methoxyphenyl)-5"-benzimidazolyl}-5'-benzimidazolyl]benzimidazole (TBZ) in the preparation or a medicament for radioprotection.

Even further according to the invention, there is provided the compound 5-(4-methylpiperazine-1-yl)-2-[2'-(2"-(4-hydroxy-3-methoxyphenyl)-5"-benzimidazolyl}-5'-benzimidazolyl]benzimidazole (TBZ).

5-Cyano-2 [3-methoxy-4-hydroxy benzimidazole] 5'-yl is produced by reacting 3,4-Diamino benzonitrile and 3-methoxy-4-hydroxy benzaldehyde. 5-Cyano-2 [3-methoxy-4-hydroxy benzimidazole] 5'-yl is then treated with Raney nickel in presence of formic acid to produce 5-Formyl-[3-methoxy-4-hydroxy benzimidazole]

The complete reaction is shown in the accompanying reaction scheme.

The pharmacological activities of two benzimidazoles having bisubstituted phenyl ring are described. The relative pharmacological activity of **5-(4 methylpiparazin-1-yl)-2-[2'-(3,4-dimethoxyphenyl)-5'-benzimidazolyl] benzimidazole (DMA) and 5-(4-methylpiperazin-1-yl)-2-[2'{2"-(4-hydroxy3methoxyphenyl) 5"benzimidazolyl} -5'-benzimidazolyl] benzimidazole (TBZ)** was compared to that of Hoechst 33342. The two compounds on the basis of earlier observation that Hoechst 33342 is more cytotoxic than Hoechst 33258 as the para phenolic group of Hoechst 33258 is being replaced by ethoxy group in Hoechst 33342. Keeping above observations in mind, we have introduced two methoxy group in the bisbenzimidaole and one methoxy and one hydroxyl goup in trisbenzimidazole. The synthesis of bisbenzimidazole was carried out basically as described by Kelly et al (1994) using 2-Amino-4-[5'-(4"-Methylpiperazin-1"-yl)benzimidazol-2'-yl]aniline and 3,4-dimethoxy benzaldehyde in 30% yield This methodology is different from the method described by Lown et al. where they have condensed 2-arylbenzimidazole with o-arylenediamine. The terbenzimidazole was prepared using a methodology described by Edmond J- La Voie (1995) using an equimolar mixture of 2-Amino-4-[5'-(4"-Methylpiperazin-1"-yl)benzimidazol-2'-yl]aniline and 5-Formyl-[3-methoxy-4-hydroxy benzimidazole] in nitrobenzene at 140-150°C for 36 h in 25% yield. In this reaction the initially formed schiff base undergoes oxidative cyclization by nitrobenzene to give terbenzimidazole. However, we introduced two important modifications in bisbenzimidazole and terbenzimidazole. This is the first report of synthesis of terbenzimidazole having bisubstitution on the phenyl ring. In the bisbenzimidazole the two methoxy groups are introduced which are electron donating whereas in the terbenzimidazole one methoxy group was replaced by hydroxyl group to observe the stability of DNA-drug complex using hydroxy group capable of hydrogen bonding.
Both of the above mentioned compounds are non-cytotoxic even at 100µM concentration even upto 72 hours after treatment in human glioma cell line BMG-1. Cell survival assay showed that 5-(4-methylpiperazin-1-yl)-2-[2'-(3,4-dimethoxyphenyl)-5'-benzimidazolyl] benzimidazole **(DMA)** has 73% cell survival at 100µM concentration whereas5-(4-methylpiperazin-1-yl)-2-[2'{2"-(4-hydroxy-3methoxyphenyl) 5"benzimidazolyl}-5'-benzimidazolyl] benzimidazole **(TBZ)** at lower concentration i.e., 10µM has shown increase in growth whereas at 100µM showed 92% cell survival. These results are further supported by growth kinetics.
Under the same conditions, the cells were irradiated with 2Gy, 5Gy and 10Gy of radiation and physico-chemical studies like UV-Vis spectroscopy, fluorescence spectroscopy and thermal denaturation studies were done. Cytotoxicity, cell survival assays were performed in vivo in human brain glioma cell line (BMG-1). The studies revealed that the bisubstituted minor groove binding analogues of Hoechst 33258 showed high degree of protection against high doses of radiation also.

The cytotoxic effect of DMA and TBZ on exponentially growing tumor cells BMG-1 was studied as a function of time. Cells were treated for 1 h with the ligands and allowed to grow for 24, 48 and 72 hours. The MTT assay was performed at the specified time points. It was observed that Hoechst 33342 was highly cytotoxic at 10µM concentration whereas DMA and TBZ do not show any cytotoxicity even at 100µM concentration. There seemed to be no effect on the metabolic status of the treated cells. However, strikingly the metabolic activity of the treated cells seemed to be enhanced as compared to the untreated control cells. To further support our results we have performed cell survival assay at 0.1uM, 10uM and 100uM concentration of drug having control as untreated cell. These experiments were done thrice in six sets. Exponentially growing cells were used in these experiments and cell survival was studied using Macrocolony assay. The plating efficiency was nearly 77%. The parent compound Hoechst 33342 showed a significant effect on cell survival. At low concentrations, Hoechst 33342 do not seem to have considerable effect on cell survival whereas at 10µM concentration the surviving fraction reached remained 45% and at 100µM only 10% of cells survived. DMA at low concentration (0.1,1µM) did not show any effect on survival but at 10µM there is a slight (3%) decrease in surviving fraction and at 100µM, 73% of the cells survived. TBZ had a significantly different behavior than the two ligands. At low concentrations (upto 10µM) , TBZ seemed to have a slight stimulatory effect on growth whereas at 100µM showed a little (9%) decrease in surviving fraction. The effect of DMA and TBZ on proliferation of exponentially growing cells at 100µM concentration was also studied The growth kinetics of the treated cells did not differ from the untreated cells. The increase in cell number as a function of time for the control as well as the treated cells remained same. The radioprotective effect of the newly synthesized ligands were studied spectroscopically as well as in human brain glioma cell line BMG-1. The spectroscopic studies done included UV-Vis sepctroscopy, Fluorescence spectroscopy, Anisotropy measurements and Lifetime measurements and Thermal denaturation studies. Calf thymus DNA was chosen as the DNA material and ligands were incubated with the DNA and irradiated at different doses of radiation. The irradiated samples did not show any difference in the measurements as compared to the control DNA sample. The degree of DNA helix stabilization is considerably increased in the presence of the radiation and thus no DNA strand breaks were detected. At the cellular level also, the ligands were incubated with the cells and cell survival assay was performed to study the radioprotective effect of the new ligands in comparison to the parent molecule Hoechst 33342. The new ligands showed high degree of radioprotection even at lower concentrations of the ligands.

The property of being non-cytotoxic coupled with the radioprotective effect of the new ligands make them potential candidate for protection against radiation.

### Examples

### Example 1:

**2-Nitro-5-chloroaniline** : A solution of metallic sodium(0.012 g, 0.52mmol) in 44ml of absolute methanol was added to 2-nitro-5-chloro acetanilide (4.8 g, 23mmol). The solution was boiled under reflux for 3h and then the solvent was evaporated to obtain the desired yellow crystalline product in 98% yield. m. p. 126-128° C

### Example 2 :

**5-(4'-methylpiperazine-1'yl)-2-nitroaniline**: A stirred mixture of 2-nitro-5-chloroaniline(3.4g, 19.76mmol), 1-methylpiperazine (4.4ml, 52mmol), anhydrous K₂CO₃ and dry DMF (41ml) was protected with a CaCl₂ drying tube and heated at 120°C for 20h. Upon cooling the mixture was poured into 300ml ice cold water and filtration of the resulting suspension afforded a yellow solid. Recrystallized using CCl₄ to give lustrous bright yellow plates to get 4.237g (100% yield) of the title compound.
m. p.: 152°C IR : ¹H NMR : δ 2.27(s,3H , NCH₃), 2.47( m,4H,H 3',5'), 6 (d, J 3Hz, 1H, H6), 6.37 (dd, J10, 3Hz, 1H, H4), 6.89(s, 2H, NH₂)

### Example 3 :

**2-Amino-4-(4`-methylpiperazzin-1'-yl) aniline** : A solution of 5-(4'-methylpiperazin-1'yl)-2-nitroaniline (2.275g, 9.63mmol ) in methanol/ethyl acetate (20:80, 98 ml) was treated with 5% Pd/C ( 500 mg) and the mixture was hydrogenated at room temperature and atmospheric pressure. After the reaction is complete , the solution is colorless. Filtration (celite) and concentration of the filtrate without delay afforded the pale yellow colored diamine (4a) in 100% yield.

### Example 4 :

**4-Amino-3-nitrobenzonitrile**: 4-cyano-2-nitroacetanilide (3g,15mmol) was heated under reflux in 10% H₂SO₄ for 30 min. Upon cooling the resulting precipitate was filtered off and dried. Recrystallization of this material (methanol/water) gave yellow crystals of 4-amino-3-nitro benzonitrile in 95% yield m p. 160°C

### Example 5:

**Ethyl-4-Amino-3-nitrobenzenecarboximidate hydrochloride :** 4-amino-3-nitrobenzonitrile (2g ,12.5mmol) was suspended in dry ethanol (200ml at 10mg/ml concentration)and cooled in an ice/water bath. Anhydrous HCl gas was bubbled rapidly through the mixture for 30-45 min. During this time, dissolution followed by rapid precipitation was observed. The vessel was fitted with a CaCl₂ drying tube and the thick suspension was stirred overnight. The ethanol was removed by rotary evaporation and the pasty residue was triturated with dry diethyl ether. Filtration, followed by drying under reduced pressure afforded the title imino ether hydrochloride as a hygroscopic bright yellow powder in 80 % yield. m. p. 233°C; IR : 3469, 3382,3340, 2227, 1631, 1556, 1271, 920, 821, 765 cm⁻¹ ;¹H NMR : δ 1.46( t, J 7Hz,3H, OCH₂CH₃), 4.58 (q, J7Hz, 3H, OCH₂CH₃), 7.16( d, J 9Hz, 2H, H6), 8.79( d, J 2Hz, 1H, H2)

### Example 6 :

**4-[5'-(4"-methylpiperazin-1-yl)benzimidazoly-2'-yl]-2-nitroaniline** : A mixture of freshly prepared 2-Amino-4-(4'-methylpiperazin-1'-yl) aniline (2.01 g, 9.75mmol) and Ethyl-4-Amino-3-nitrobenzenecarboximidate hydrochloride (1.98 g, 9.5mmol) in dry ethanol/ glacial acetic acid (2:1,69 ml) was maintained under nitrogen and heated at reflux for 4 h. The mixture was cooled and then concentrated to afford an orange pasty residue. This material was dissolved in water and addition of conc. Ammonia solution resulted in precipitation of a solid; the suspension was allowed to stand overnight The brick red solid was collected by filtration and washed thoroughly with water before being dissolved in acetic acid/ methanol (7.5: 92.5), 50 ml. The deep red solution was filtered and then made alkaline with conc. Ammonia solution (c. 20ml) .A fine orange precipitate formed immediately and the suspension was allowed to stand for several hours before filtration. The solid thus retained was washed thoroughly with water and then with acetone. Drying under reduced pressure afforded an orange solid in 74% yield m. p. 184-186° C;
IR ; 3494, 3365, 2939, 2808, 1639, 1510, 1247, 796 cm ⁻¹;¹H NMR : δ 2.99(s,3H, NCH₃), 3.16 (m,2H), 3.34 (m,2H), 3.67 (m,2H), 3.94 (m,2H), 7.24 (d,J9Hz,1H), 7.25(d, J2Hz, 1H;), 7.36 (dd, J9Hz, 1H), 7.98 ( dd, J 9.2 Hz, 1H), 8.95 (d, J 2Hz, 1H)

### Example 7:

**2-Amine-4-[5'-(4"-Methylpiperazin-1"-yl)benzimidazol-2'-yl]aniline :**
A solution of 4-[5'-(4"-methylpiperazin-1"-yl)benzimidazol-2'-yl]-2-nitroaniline (1.04 g ,30mmol) in ethyl acetate/methanol (80 ml of 2:1 mixture) was treated with 5% palladium on carbon (250mg) and hydrogenated at room temperature and atmospheric pressure. When hydrogen uptake has ceased , the solution was filtered (celite) and concentrated without delay to afford the orange-brown colored diamine .

### Example 8 :

**5-Cyano-2 [3-methoxy-4-hydroxy benzimidazole] 5'-yl :** A solution of 1.224 g (9.2 mmol) of previously obtained 3,4-Diamino benzonitrile , 1.4 g (9.2mmol) of 3-methoxy-4-hydroxy benzaldehyde in nitrobenzene are taken in a three necked round bottom flask under nitrogen and heated at 140° C. The reaction mixture is heated for 18 hours with stirring, nitrobenzene is then removed under reduced pressure to obtain the brown colored crude product (14). The final product is then obtained through silica gel (60-120 mesh size) column chromatography using EtOAc / MeOH as eluent m.p. 226-230° C; IR 3425.3 (O-H), 3263.3 (-NH), 2221.8 (-CN), 1278.7 (C-O-C) cm⁻¹; ¹H NMR δ 13.5 (B, 1H, -NH), 9.52 ( s, 1H, -OH),7.85 (d, 1H, C7), 7.82 (d, 1H, C6), 7.72 (s, 1H, C3'),7.63 (d,1H,C5'), 6.93 (d,1H, C6'), 3.90 (s,3H, -OCH₃)

### Example 9 :

**5-Formyl-[3-methoxy-4-hydroxy benzimidazole] :** To a solution of 1g (3.77mmol) 5-Cyano-2 [3-memoxy-4-hydroxy benzimidazole] 5'-yl in 60ml formic acid and 20 ml of water, Raney nickel (3.96 g) was added. The reaction mixture was heated at 95° C for 6 hours. The hot mixture was filtered (celite) and the reaction flask and the celite bed were rinsed with water. The aqueous solution was concentrated to dryness. To this residue, water is added to obtain a white precipitate. The pH of this suspension was adjusted to 9 by the dropwise addition of 2 N NaOH. The product was obtained by extraction with ethyl acetate. The ethyl acetate extract was dried (Na₂SO₄) and concentrated in vacuo to give the yellow colored compound in 30% yield. m. p. 258 -260°C ; IR 3435.6 (-OH) ,3194.55 (NH), 1675.6 (CHO), 1594, 1506, 1441.6, 1280.7cm⁻¹ ; ¹H NMR δ 13.7 (b, 1H, -NH), 10.2 (S,1H, -CHO) ,9.5 (s,1H, -OH), 8.25 (d,1H, C6), 7.78 (s,1H, C4), 7.75 (d,1H, C7), 7.7 (s,1H, C3'), 7.65 (d,1H, C5'), 6.92 (d,1H, C6'), 3.92 (s,3H, -OCH₃)

### Example 10 :

**5-(4-methylpiperazin-1-yl)-2-[2'-(3,4-dimethoxyphenyl)-5'-benzimidazolyl] benzimidazole** : A solution of freshly prepared 2-Amino-4-[5'-(4"-Methylpiperazine-1"-yl)benzimidazol-2'-yl]aniline (1.18g,3.67mmol) and 3,4-dimethoxy benzaldehyde ( 0.61g ,3.67mmol) in nitrobenzene (110 ml) is heated at 140-150° C for 24 h. The solvent is then removed under reduced pressure to give the final crude product as a brown colored solid. The product is purified by column chromatography on BUCHI 688 Liquid (MPLC) Pump using silica gel (70-230 mesh size) and EtOAc/ MeOH as eluent to give a yellow colored compound and characterized by spectroscopic techniques .m. p. 220°C Yield : 30%; IR : 3556, 2922, 1629, 1508, 1417, 1371, 1296, 1022, 810cm⁻¹; ¹H NMR (DMSO-d₆) δ 2.24 (s,3H,NCH₃), 2.46 (t,4H,CH₂,J=4Hz), 3.5 (t,4H,CH₂, J=4Hz), 3.86 (s,3H,OCH₃), 6.77 (d,1H,Ar-h,J=8Hz), 7.13 (d,2H,Ar-H, J= 9Hz), 7.64 (d,1H,Ar-H,J=8.5Hz ), 7.78 (d,1H,Ar-H,J=8 Hz), 8.03 (m,1H,Ar-H),8.17(d,2H,Ar-h, J=9Hz), 8.3 (s,1H,Ar-H), 13.0 (bs,2H,NH) .D₂O exchange resulted in the disappearance of the peak at δ13.0.; EIMS : 467 (M⁺), 425,411,261,235,220,194,118,91,55,44

### Example 11 :

**5-(4-methylpiperazin-1-yl)-2-[2'{2"-(4-hydroxy-3methoxyphenyl)5"benzimidazolyl} -5'-benzimidazolyl] benzimidazole** : A solution of freshly prepared 2-Amino-4-[5'-(4"-Methylpiperazin-1"-yl)benzimidazol-2'-yl]aniline (218 mg, 0.82mmol) and 5-Formyl-[3-methoxy-4-hydroxy benzimidazole] (182 mg ,0.68mmol) in nitrobenzene is taken under argon in a round bottom flask and heated at 140°C for 20 hours. Nitrobenzene is removed under reduced pressure and the resulting solid is purified by column chromatography (EtOAc/ MeOH) on BUCHI 688 Liquid (MPLC) Pump using silica gel (70-230 mesh size) to obtain the final product (25% yield) as a brown colored solid. m.p. >290°C ; IR 3435, 3195, 1632, 1560, 1413, 1281; ¹H NMR δ 13.4-13.55 ( b,3H ), 9.5 (s,1H), 8.45 (s, 1H ), 8.39 (s, 1H), 8.03 - 8.08 (m,4H), 7.9 (d,1H), 7.68 (d,1H), 7.38 (d,1H), 6.91-6.98 (m,3H), 3.92 (s, 3H), 3.23 (t,4H), 2.68 (t, 4H), 2.60 (s,3H) The mass of TBZ was observed on MALDI: 570.3

### Cytotoxicity Assay

The cytotoxicity was determined using the MTT microtiter plate tetrazolium cytotoxicity assay (MTA). The human brain malignant glioma cell line BMG-1 was used. The cytotoxicity assay was performed using 96-well microtiter plates. 3000 cells/ well were plated and treatment of the ligands was done 24 hours post plating. For determination of IC₅₀ , cells were exposed continuously with varying concentration of drug and MTT assays were performed at the end of fourth day. To the control and treated cells , 20 µl of 5mg/ml MTT in PBS was added, incubated for 2 hours at 37°C and then the medium was removed. 150 µl of DMSO was added and the plate was read at 540 nm with reference at 630 nm.

### Macrocolony Assay

Cells were washed with HBSS and harvested using 0.05% trypsin. Depending on the treatments, 200-1200 cells were plated in 90-mm Petri dishes and incubated at 37°C in a 5% CO₂ humidified atmosphere for 8-10 days. Colonies were fixed in methanol and stained with 1% crystal violet. Colonies containing more than 50 cells were counted as shown in Table 1&2.
**Proliferation Kinetics** BMG-1 cells were seeded at 7000-8000 cells/ cm², and their proliferation kinetics was measured at 24-h intervals by trypsinizinig and counting total cells per flask using a hemocytometer.

**Table 1**

| **Thermal Denaturation studies of ligand-DNA complex in presence and absence of radiation** | | | |
|---|---|---|---|
| Ligand | Ratio [ligand]/[DNA] | Dose (Gy) | Tm (°C) |
| TBZ | | 0 | 74.3 |
| | 0.01 | 100 | 75 |
| | | 0 | 77.7 |
| | 0.1 | 100 | 81.5 |
| Hoechst 33258 | | 0 | 71.5 |
| | 0.01 | 100 | 67.5 |
| | | 0 | 81.7 |
| | 0.1 | 100 | 79.3 |

**Table 2**

| **Cell Survive Assay in BMG-1 cells with and without irradiation** | | |
|---|---|---|
| Treatment | Plating efficiency % | Survival fraction |
| Control | 67 | 1 |
| DMA (1uM) | 65 | 0.97 |
| DMA(10uM) | 67 | 1 |
| DMA (100uM) | 64 | 0.95 |
| TBZ (1uM) | 73 | 1.07 |
| TBZ (10uM) | 71 | 1.05 |
| TBZ (100uM) | 69 | 1.02 |
| 2Gy | 51 | 0.75 |
| 5Gy | 15 | 0.22 |
| 10Gy | 2.0 | 0.03 |
| DMA (10uM)+2Gy | 63 | 0.93 |
| TBZ (10uM) + 2Gy | 60 | 0.89 |
| DMA (1uM) +5 Gy | 22 | 0.32 |
| DMA(10uM)+5Gy | 27 | 0.40 |
| DMA (100uM) + 5 Gy | 25 | 0.38 |
| TBZ 1uM) + 5Gy | 21 | 0.31 |
| TBZ (10uM) + 5Gy | 20 | 0.30 |
| TBZ (100uM) + 5 Gy | 18 | 0.26 |
| DMA (10uM) + 10 Gy | 4 | 0.06 |
| TBZ (10uM) +10 Gy | 3.5 | 0.05 |

### Experiment with Normal human embryonic kidney cell line (Hek cell line):

To demonstrate the effect of DMA and TBZ as radioprotectors in comparison to Hoechst 33342, we also chose a normal (untransformed) human embryonic kidney cell line. Though the two cell lines, Hek and BMG-1 cell line are not radiobiologically different, it was still reasonable to demonstrate the *invitro* effect of these ligands on different cell lines.

The results with the two cell lines are not significantly different but it can be concluded that both DMA and TBZ are better radioprotectors in comparison to Hoechst 33342.

Below presented are the results of the growth kinetics and Macrocolony assay performed with the two cell lines.

### Procedure:

### Macrocolony Assay (Cell Survival Assay)

Both cell lines in triplicate were washed with HBSS, plated and grown for four days before harvesting using 0.05% trypsin, Depending on the treatments, 200 to 1200 cells were plated in 90 mm petri dishes and incubated at 37°C in 5% CO₂ humidified atmosphere for 8 to 10 days. Colonies were fixed with methanol and stained with 1 % crystal violet Colonies containing more than 50 cells were counted.

### Proliferation Kinetics

Both the cell lines in triplicates were seeded at 7000 to 8000 cells/ cm², and their proliferation kinetics was studied at 24 h intervals following trypsinization and counting total cells per flask using a haemocytometer.

### Irradiation Procedures:

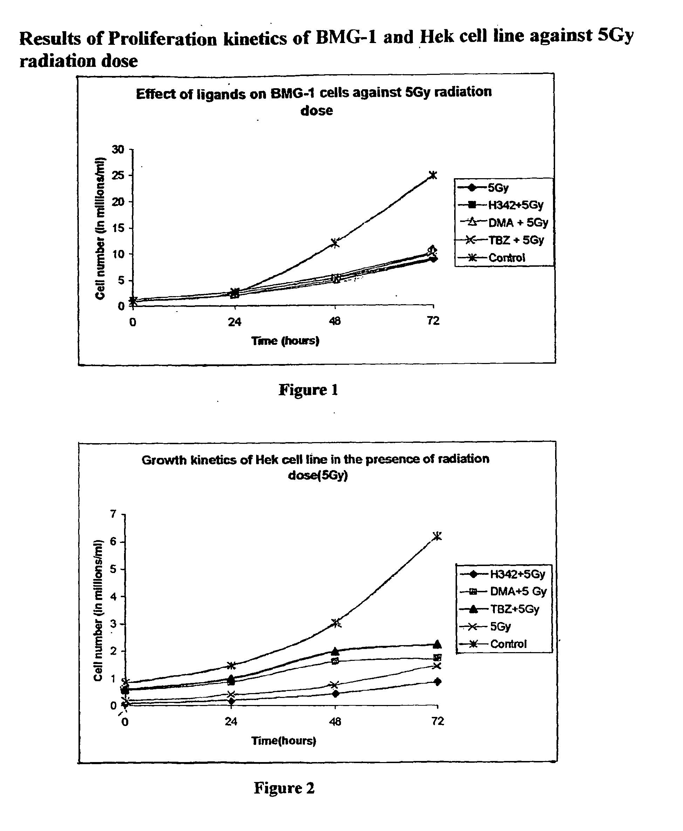

Exponentially growing cells, 24 h after plating were irradiated at room temperature in growth medium with a Co-60 source (Gamma cell, AECL, Canada) at a dose rate of 0.5 to 1.2 Gy/min. Concentration of 10µm drug was added 1h before irradiation. Following this procedure, cells were grown for various time intervals to study growth kinetics and plated for Macrocolony assay as shown in the following Tables 3 & 4 and figure 1 & 2

**Table 3**

| **Cell Survival Assay in BMG-1 cells with 5 Gy irradiation** | | |
|---|---|---|
| Treatment | Plating efficiency % | Survival fraction |
| Control | 67 | 1 |
| 5Gy | 15 | 0.22 |
| DNA (10uM) + 5 Gy | 27 | 0.40 |
| TBZ (10uM) + 5 Gy | 20 | 0.30 |
| | | |
| Hoechst 33342 + 5Gy | 20 | 0.10 |

**Table 4**

| **Cell Survival Assay in Hek cells with 5Gy irradiation** | | |
|---|---|---|
| Treatment | Plating efficiency % | Survival friction |
| Control | 70 | 1 |
| 5Gy | 15 | 0.22 |
| DMA (10uM) + 5Gy | 27 | 0.50 |
| TBZ (10uM) + 5 Gy | 25 | 0.48 |
| Hoechst 33342 + 5Gy | 20 | 0.10 |

## Claims

1. A process for the synthesis of bisbenzimidazoles and its derivations comprising:
(i) reacting 5-chloroaniline with zinc dust and acetic anhydride to produce 5-chloroacetanilide;
(ii) reacting 5-chloracetanilide with HNO₃ to produce 2-nitro-5-chloroacetanilide;
(iii) adding sodium methoxide to 2-nitro-5-chloroacetanilide to produce 2-nitro-5-chloroaniline;
(iv) heating 2-nitro-5-chloroaniline, methyl piperazine, anhydrous K₂CO₃ and dimethyl formamide at 100 to 120 °C to produce a mixture which is cooled by pouring ice and is filtered to obtain 5-(4'-methylpiperazin-1'-yl)-2- nitroaniline;
(v) treating 5-(4'-methylpiperazin-l'-yl)-2-nitroaniline with Pd/C to produce 2-amino-4-(4'-methylpiperazin-1'-yl)aniline;
(vi) refluxing a mixture of 2-amino-4-(4'-methylpiperazin-1'-yl)aniline and ethyl-4-amino-3-nitrobenzenecarboximidate hydrochloride in the presence of ethanol/glacial acetic acid to produce 4-[5'-(4"-methylpiperazin-1"-yl)benzimidazol-2'-yl]-2-nitroaniline;
(vii) treating a solution of 4-15'-(4"-methylpiperazin-1"-yl)benzimidazol-2'-yl]-2-nitroaniline with palladium on carbon to yield 2-amino-4-[5'-(4"-methylpiperazin-1"-yl)benzimidazol-2'-yl]aniline; and
(viii) heating 2-amino-4-[5'-(4"-methylpiperazin-1"-yl)benzimidazol-2'-yl]aniline and 3-4-dimethoxybenzaldehyde using nitrobenzene as a solvent at 110 to 150 °C to produce 5-(4-methylpiperazin-1-yl)-2-[2'-(3,4-dimethoxyphenyl)-5'-benzimidazolyl]benzimidazole (DMA); or
(ix) heating 2-amino-4-[5'-(4"-methylpiperazin-1"-yl)benzimidazol-2'-yl]aniline and 5-formyl-[3-methoxy-4-hydroxy benzimidazole] using nitrobenzene at 110 to 150 °C in the presence of argon to produce 5-(4-methylpiperazine-1-yl)-2-[2'-{2"-(4-hydroxy-3-methoxyphenyl)-5"-benzimidazolyl}-5'-ben/midazolyl]benzimidazole (TBZ).

2. 5-(4-methylpiperazin-1-yl)-2-[2'-(3,4-dimethoxyphenyl)-5'-benzimidazolyl]benzimidazole (DMA) or 5-(4-methylpiperazine-1-yl)-2-[2'-{2"-(4-hydroxy-3-methoxyphenyl)-5"-benzimidazolyl}-5'-benzimidazolyl]benzimidazole (TBZ) for use as a radioprotecting agent.

3. Use of 5-(4-methylpiperazin-1-yl)-2-[2'-(3,4-dimethoxyphenyl)-5'-benzimidazolyl]benzimidaxole (DMA) or 5-(4-methylpiperazine-1-yl)-2-[2'-{2"-(4-hydroxy-3-methoxyphenyl)-5"-benzimidazolyl}-5'-benzimidazolyl]benzimidazole (TBZ) in the preparation of a medicament for radioprotection.

4. The compound 5-(4-methylpiperazine-1-yl)-2-[2'-{2"-(4-hydroxy-3-methoxyphenyl)-5"-benzimidazolyl}-5'-benzimidazolyl]benzimidazole (TBZ).

## Patentansprüche

1. Verfahren zur Synthese von Bisbenzimidazolen und ihren Derivaten, umfassend:
(i) das Reagieren von 5-Chloranilin mit Zinkstaub und Essigsäureanhydrid un t e r Bildung von 5-Chloracetanilid;
(ii) das Reagieren von 5-Chloracetanilid mit HNO₃ unter Bildung von 2-Nitro-5-chloracetanilid;
(iii) das Hinzugeben von Natriummethoxid zu 2-Nitro-5-chloracetanilid unter Bildung von 2-Nitro-5-chloranilin;
(iv) das Erhitzen vo n 2-Nitro-5-chloranilin, Methylpiperazin, wasserfreiem K₂CO₃ und Dimethylformamid bei 100 bis 120 °C unter Bildung einer Mischung, die durch Gießen von Eis gekühlt und filtriert wird, um 5-(4'-Methylpiperazin-1'-yl)-2-nitroanilin zu erhalten;
(v) das Behandeln von 5-(4'-Methylpiperazin-1'-yl)-2-nitroanilin mit Pd/C unter Bildung von 2-Amino-4-(4'-methylpiperazin-1'-yl)anilin;
(vi) das Rückflusskochen einer Mischung von 2-Amino-4-(4'-methylpiperazin-1'-yl) anilin un d Ethyl-4-amino-3-nitrobenzolcarboximidathydrochlorid in Gegenwart von Ethanol/Eisessig unter Bildung von 4-[5'-4"-Methylpiperazin-1"-yl)benzimidazol-2'-yl]-2-nitroanilin;
(vii) das Behandeln einer Lösung von 4-[5'-(4"-Methylpiperazin-1"-yl)benzimidazol-2'-yl]-2-nitroanilin mit Palladium auf Kohlenstoff, um 2-Amino-4-[5'-(4"-methylpiperazin-1"-yl)benzimidazol-2'-yl]anilin zu ergeben; und
(viii) das Erhitzen vo n 2-Amino-4-[5'-(4"-methylpiperazin-1"-yl)benzimidazol-2'-yl]anilin und 3-4-Dimethoxybenzaldehyd unter Verwendung von Nitrobenzol als Lösungsmittel bei 110 bis 150 °C unter Bildung von 5-[4-Methylpiperazin-1-yl)-2-[2'-(3,4-dimethoxyphenyl)-5'-benzimidazolyl]benzimidazol (DMA); oder
(ix) das Erhitzen vo n 2-Amino-4-[5'-(4"-methylpiperazin-1"-yl)benzimidazol-2'-yl]anilin u n d 5-Formyl-[3-methoxy-4-hydroxybenzimidazol] unter Verwendung von Nitrobenzol bei 110 bis 150 °C in Gegenwart von Argon unter Bildung von 5-[4-Methylpiperazin-1-yl)-2-[2'-{2"-(4-hydroxy-3-methoxyphenyl)-5"-benzimidazolyl}-5'-benzimidazolyl]benzimidazol (TBZ).

2. 5-[4-Methylpiperazin-1-yl)-2-[2'-(3,4-dimethoxyphenyl)-5'-benzimidazolyl]benzimidazol (DMA) oder 5-[4-Methylpiperazin-1-yl)-2-[2'-{2"-(4-hydroxy-3-methoxyphenyl)-5"-benzimidazolyl}-5'-benzimidazolyl]benzimidazol (TBZ) zur Verwendung als Radioschutzmittel.

3. Verwendung von 5-[4-Methylpiperazin-1-yl)-2-[2'-(3,4-dimethoxyphenyl)-5'-benzimidazolyl]benzimidazol (DMA) oder 5-[4-Methylpiperazin-1-yl)-2-[2'-{2"-(4-hydroxy-3-methoxyphenyl)-5"-benzimidazolyl}-5'-benzimidazolyl] benzimidazol (TBZ) bei der Herstellung eines Medikaments zum Radioschützen.

4. Die Verbindung 5-[4-Methylpiperazin-1-yl)-2-[2'-{2"-(4-hydroxy-3-methoxyphenyl)-5"-benzimidazolyl}-5'-benzimidazolyl]benzimidazol (TBZ).

## Revendications

1. Procédé pour la synthèse de bisbenzimidazoles et de leurs dérivés comprenant :
(i) la réaction de 5-chloroaniline avec de la poussière de zinc et de l'anhydride acétique pour produire du 5-chloroacétanilide ;
(ii) la réaction de5-chloroacétanilide avec HNO₃ pour produire du 2-nitro-5-chloroacétanilide ;
(iii) l'ajout deméthylate de sodium à du2-nitro-5-chloroacétanilide pour produire de la 2-nitro-5-chloroaniline ;
(iv) le chauffage de 2-nitro-5-chloroaniline, de méthylpipérazine, de K₂CO₃ anhydre et dediméthylformamide à 100 à 120 °C pour produire un mélange qui est refroidi par versement de glace et filtré pour obtenir de la 5-(4'-méthylpipérazin-1'-yl)-2-nitroaniline ;
(v) le traitementde 5-(4'-méthylpipérazin-1'-yl)-2-nitroaniline avec du Pd/C pour produire de la 2-amino-4-(4'-méthylpipérazin-1'-yl)aniline ;
(vi) le chauffage au reflux d'un mélange de 2-amino-4-(4'-méthylpipérazin-1'-yl)aniline et de chlorhydrate de 4-amino-3-nitrobenzènecarboximidate d'éthyle en présence d'éthanol/acide acétique glacial pour produire d e la 4-[5'-(4□-méthylpipérazin-1□-yl)benzimidazol-2'-yl]-2-nitroaniline ;
(vii) le traitement d'une solution de 4-[5'-(4□-méthylpipérazin-1□-yl)benzimidazol-2'-yl]-2-nitroaniline avec du palladium sur du carbone pour produire de la 2-amino-4-[5'-(4□-méthylpipérazin-1□-yl)benzimidazol-2'-yl]aniline ; et
(viii) 1 e chauffage de 2-amino-4-[5'-(4□-méthylpipérazin-1□-yl)benzimidazol-2'-yl]aniline et de 3,4-diméthoxybenzaldéhyde à l'aide de nitrobenzène en tant que solvant à 110 à 150 °C pour produire du 5-(4-méthylpipérazin-1-yl)-2-[2'-(3,4-diméthoxyphényl)-5'-benzimidazolyl]benzimidazole (DMA) ; ou
(ix) 1 e chauffage de 2-amino-4-[5'-(4□-méthylpipérazin-1□-yl)benzimidazol-2'-yl]aniline e t de 5-formyl-[3-méthoxy-4-hydroxybenzimidazole] à l'aide de nitrobenzène à 110 à 150 °C en présence d'argon pour produire du 5-(4-méthylpipérazin-1-yl)-2-[2'-{2□-(4-hydroxy-3-méthoxyphényl)-5□-benzimidazolyl}-5'-benzimidazolyl]benzimidazole (TBZ).

2. 5-(4-Méthylpipérazin-1-yl)-2-[2'-(3,4-diméthoxyphényl)-5'-benzimidazolyl]benzimidazole (DMA) ou 5-(4-méthylpipérazin-1-yl)-2-[2'-{2□-(4-hydroxy-3-méthoxyphényl)-5□-benzimidazolyl}-5'-benzimidazolyl]benzimidazole (TBZ) destiné à être utilisé comme agent radioprotecteur.

3. ^tilisation de 5-(4-méthylpipérazin-1-yl)-2-[2'-(3,4-diméthoxyphényl)-5'-benzimidazolyl]benzimidazole (DMA) ou de 5-(4-méthylpipérazin-1-yl)-2-[2'-{2□-(4-hydroxy-3-méthoxyphényl)-5□-benzimidazolyl}-5'-benzimidazolyl]benzimidazole (TBZ) dans la préparation d'un médicament pour la radioprotection.

4. Composé 5-(4-méthylpipérazin-1-yl)-2-[2'-{2□-(4-hydroxy-3-méthoxyphényl)-5□-benzimidazolyl}-5'-benzimidazolyl]benzimidazole (TBZ).
